# EUROPEAN PATENT APPLICATION

(11) **EP 3 747 485 A1**
(43) Date of publication of application: **09.12.2020**
(21) Application number: 19778245.1
(22) Date of filing: 07.02.2019
(51) Int. Cl.: A61M 5/168, A61M 5/14, A61M 5/145

(54) **INFUSION DEVICE AND SYRINGE PUMP**

(30) Priority: 29.03.2018 JP 2018065569
(71) Applicant: Terumo Kabushiki Kaisha, Tokyo 151-0072 (JP)
(72) Inventor: NOMURA Takafumi, Ashigarakami-gun, Kanagawa 259-0151 (JP)
(74) Representative: Casalonga
(86) International application number: PCT/JP2019/004481
(87) International publication number: WO 2019/187690

(57) **Abstract**

An infusion device includes an infusion bag configured to contain a first fluid, a syringe pump configured to hold a syringe and be capable of delivering a second fluid contained in the syringe to an infusion line connected to the infusion bag, and a fluid sensor configured to be capable of transmitting a predetermined signal when a residual volume of the first fluid contained in the infusion bag is equal to or less than a predetermined volume. The syringe pump includes a communication unit configured to be capable of receiving the predetermined signal from the fluid sensor, a notification unit configured to be capable of notifying outside of predetermined information, and a notification control unit configured to cause the notification unit to notify of the predetermined information when the communication unit receives the predetermined signal.

## Description

### Technical Field

The present disclosure relates to an infusion device and a syringe pump.

### Background Art

In surgery performed on a living body such as a human body, an anesthetic contained in a syringe is administered to the living body by a syringe pump. Administering of such an anesthetic is typically performed by coinfusion into an infusion line that connects an infusion bag and a living body. A residual volume of the infusion bag is monitored. Every time the infusion bag becomes short of residual volume, the infusion bag is replaced with a new one. It is often the case that a medical staff visually checks an infusion bag to monitor a residual volume of the infusion bag. Furthermore, as recited in Patent Literature 1, a residual volume of an infusion bag may be monitored through, for example, a computer that can receive a signal from a fluid sensor disposed in the infusion bag. Citation List

### Patent Literature

Patent Literature 1: JP 2015-96098 A

### Summary of Invention

### Technical Problem

Even when an infusion bag becomes short of residual volume, in some case, actions for the shortage is postponed due to an unforeseen situation such as blood loss during surgery. In a case where an infusion line is used to deliver a fluid, an infusion pump can urge the replacement of the empty infusion bag with an alert from the infusion pump that notifies of the entry of air bubbles. On the other hand, in a case where a fluid is delivered through an infusion line by gravity drip, there is a possibility that the shortage of the residual volume of the infusion bag is overlooked.

However, with a shortage of residual volume inside an infusion bag, even when an anesthetic is delivered through an infusion line by a syringe pump, the reduction of flow rate inside the infusion line causes a reduced flow rate of the anesthetic to be delivered to a living body. This may cause a serious situation such as intraoperative awareness. Such a problem is also caused in a case using predetermined drugs other than anesthetics such as hypertensive drugs and hypotensive drugs.

In view of such a problem, an object of the present disclosure is to provide an infusion device and a syringe pump that can urge the replacement of an infusion bag when the infusion bag becomes short of residual volume, which can reliably achieve the intended effect of a predetermined drug administered by a syringe pump.

### Solution to Problem

An infusion device according to a first aspect of the present invention includes:
an infusion bag configured to contain a first fluid;
a syringe pump configured to hold a syringe and be capable of delivering a second fluid contained in the syringe to an infusion line connected to the infusion bag; and
a fluid sensor configured to be capable of transmitting a predetermined signal when a residual volume of the first fluid contained in the infusion bag is equal to or less than a predetermined volume,
in which the syringe pump includes:
   a communication unit configured to be capable of receiving the predetermined signal from the fluid sensor;
   a notification unit configured to be capable of notifying outside of predetermined information; and
   a notification control unit configured to cause the notification unit to notify of the predetermined information when the communication unit receives the predetermined signal.

As an embodiment of the present invention,
the syringe pump includes:
a delivery control unit configured to be capable of controlling delivery of the second fluid to the infusion line; and
a determination unit configured to determine whether the communication unit and the fluid sensor are communicably connected to each other,
the delivery control unit being configured not to start delivering the second fluid to the infusion line until the determination unit determines that the communication unit and the fluid sensor are communicably connected to each other.

As an embodiment of the present invention,
the syringe pump includes:
a delivery control unit configured to be capable of controlling delivery of the second fluid to the infusion line; and
a determination unit configured to determine whether the communication unit and the fluid sensor are communicably connected to each other and determine whether the second fluid is a predetermined drug,
the delivery control unit being configured not to start delivering the second fluid to the infusion line until the determination unit determines that the communication unit and the fluid sensor are communicably connected to each other when the determination unit determines that the second fluid is the predetermined drug.

As an embodiment of the present invention,
the fluid sensor can transmit another signal different from the predetermined signal when the residual volume of the first fluid exceeds the predetermined volume, and
the syringe pump includes a delivery control unit configured to be capable of controlling delivery of the second fluid to the infusion line,
the delivery control unit being configured not to start delivering the second fluid to the infusion line until the communication unit receives the other signal.

As an embodiment of the present invention,
the fluid sensor can transmit another signal different from the predetermined signal when the residual volume of the first fluid exceeds the predetermined volume, and
the syringe pump includes:
a determination unit configured to determine whether the second fluid is a predetermined drug; and
a delivery control unit configured to be capable of controlling delivery of the second fluid to the infusion line,
the delivery control unit being configured not to start delivering the second fluid to the infusion line until the communication unit receives the other signal when the determination unit determines that the second fluid is the predetermined drug.

As an embodiment of the present invention, the fluid sensor is an ultrasonic sensor disposed in the infusion bag.

As a second aspect of the present invention,
a syringe pump is configured to hold a syringe and be capable of delivering a second fluid contained in the syringe to an infusion line connected to an infusion bag that contains a first fluid, the syringe pump including:
a communication unit configured to be capable of receiving a predetermined signal transmitted from a fluid sensor when a residual volume of the first fluid contained in the infusion bag is equal to or less than a predetermined volume;
a notification unit configured to be capable of notifying outside of predetermined information; and
a notification control unit configured to cause the notification unit to notify of the predetermined information when the communication unit receives the predetermined signal.

### Advantageous Effects of Invention

According to the present disclosure, there is provided an infusion device and a syringe pump that can urge the replacement of an infusion bag when the infusion bag becomes short of residual volume, which can reliably achieve the intended effect of a predetermined drug administered by a syringe pump.

### Brief Description of Drawings

Fig. 1 is an external view schematically illustrating an infusion device according to a first embodiment of the present invention.
Fig. 2 is a block diagram illustrating functions of the infusion device illustrated in Fig. 1.
Fig. 3 is a flowchart illustrating an operating procedure of the infusion device illustrated in Fig. 1.
Fig. 4 is an external view schematically illustrating an infusion device according to a second embodiment of the present invention.
Fig. 5 is a block diagram illustrating functions of the infusion device illustrated in Fig. 4.

### Description of Embodiments

Hereinafter, an infusion device and a syringe pump according to an embodiment of the present invention will be described in detail with reference to the drawings.

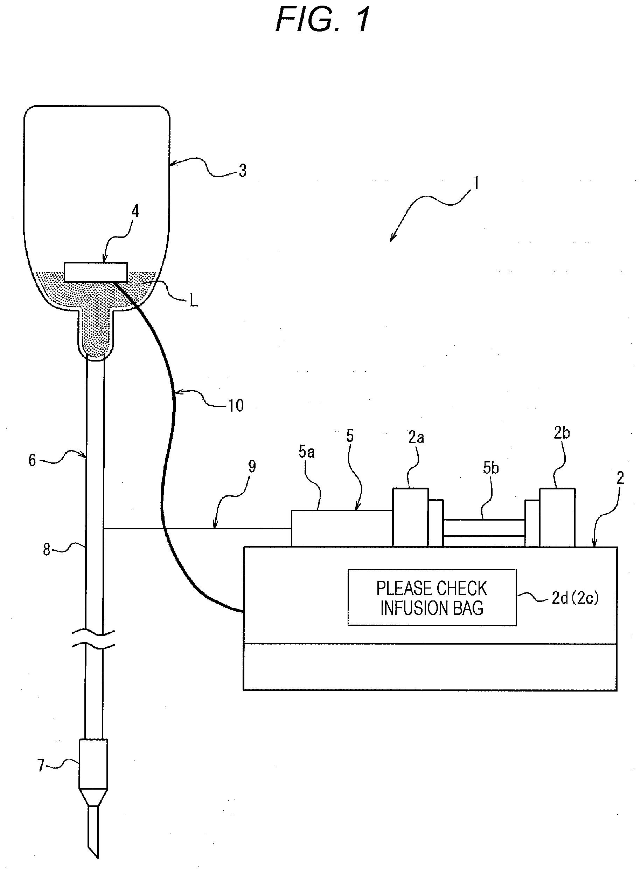

First, an infusion device 1 and a syringe pump 2 according to a first embodiment of the present invention will be described in detail with reference to Figs. 1 to 3. As illustrated in Fig. 1, the infusion device 1 according to this embodiment includes the syringe pump 2, an infusion bag 3, and a fluid sensor 4.

The syringe pump 2 is configured to hold a syringe 5 and be capable of delivering a second fluid contained in the syringe 5 to an infusion line 6 connected to the infusion bag 3. The second fluid may be a predetermined drug which may cause a serious situation when the fluid to be delivered to a living body decreases in flow rate. Examples of the predetermined drug include anesthetics, hypertensive drugs, hypotensive drugs, and the like. The syringe 5 includes a cylindrical barrel 5a and a plunger 5b. The barrel 5a partitions an ejection hole and a hollow linked to the ejection hole. The plunger 5b is to be inserted into the hollow of the barrel and pushed toward the ejection hole so that the second fluid inside the hollow is pushed out from the ejection hole. The syringe pump 2 includes a syringe clamp 2a that can fix the barrel 5a and a slider 2b that can press the plunger 5b toward the ejection hole. The syringe pump 2 also includes a display 2d included in a notification unit 2c that can notify outside of predetermined information to urge the replacement of the infusion bag 3. The display 2d may be a display panel that can show various kinds of information or may be a touch panel that can accept various kinds of input.

The infusion line 6 connects the infusion bag 3 and a living body such as a human body. The infusion line 6 includes an indwelling member 7 such as an indwelling needle to be placed inside the living body. The infusion line 6 also includes a connecting member 8 having one end connected to the infusion bag 3 and the other end connected to the indwelling member 7. The connecting member 8 may include, for example, a tube, a connector, and a coinfusion plug. The coinfusion plug included in the connecting member 8 is connected to one end of a coinfusion line 9 having the other end connected to the ejection hole of the syringe 5.

The infusion bag 3 contains a first fluid L including a predetermined component according to the intended use. In this embodiment, the first fluid L contained in the infusion bag 3 is delivered to the living body by gravity drip. However, an infusion pump may be disposed on the infusion line 6, and the first fluid L may be delivered by the infusion pump.

The fluid sensor 4 is configured to be capable of transmitting a predetermined signal (hereinafter also referred to as "first signal") when a residual volume of the first fluid L contained in the infusion bag 3 is equal to or less than a predetermined volume. Furthermore, the fluid sensor 4 is configured to be capable of transmitting another signal different from the first signal (hereinafter also referred to as "second signal") when the residual volume of the first fluid L exceeds the predetermined volume. The fluid sensor 4 in this embodiment is an ultrasonic sensor disposed in the infusion bag 3. The fluid sensor 4 is not limited to such an ultrasonic sensor and may be, for example, a capacitance sensor that can detect changes of capacitance inside the infusion bag 3. The fluid sensor 4 in this embodiment is connected to the syringe pump 2 by a cable 10. The fluid sensor 4 is attachable to and detachable from the infusion bag 3 by a double-sided tape or the like.

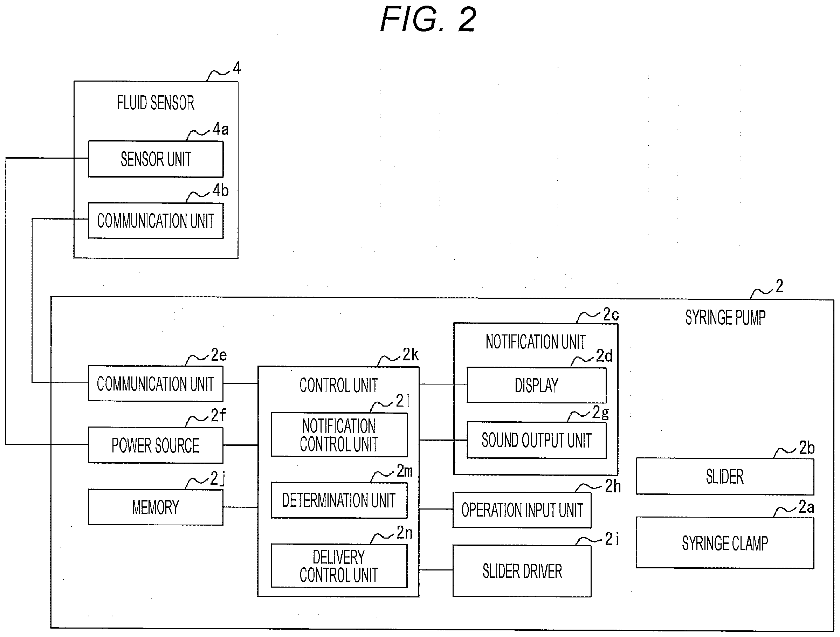

As illustrated in Fig. 2, the fluid sensor 4 includes a sensor unit 4a provided with an ultrasonic transmitter that can transmit an ultrasonic wave to the inside of the infusion bag 3 and an ultrasonic receiver that can receive a reflected wave of the ultrasonic wave transmitted from the ultrasonic transmitter. The syringe pump 2 includes a communication unit 2e that can receive the first signal from the fluid sensor 4. The fluid sensor 4 includes a communication unit 4b that can communicate with the communication unit 2e. The communication unit 2e of the syringe pump 2 can receive via the cable 10 either the first signal or the second signal from the communication unit 4b of the fluid sensor 4 depending on the residual volume of the first fluid L. The fluid sensor 4 is connected to a power source 2f disposed in the syringe pump 2 via the cable 10 and can operate by electric power from the power source 2f.

The syringe pump 2 includes the display 2d and a sound output unit 2g as components of the notification unit 2c. The display 2d can output predetermined visual information to urge the replacement of the infusion bag 3. The sound output unit 2g can output predetermined aural information to urge the replacement of the infusion bag 3. The notification unit 2c may include either the display 2d or the sound output unit 2g.

The syringe pump 2 includes an operation input unit 2h that can receive various kinds of input for making the syringe pump 2 operates. The operation input unit 2h includes, for example, operation buttons. Part or all of the operation input unit 2h may be a touch panel included in the display 2d.

The syringe pump 2 includes a slider driver 2i that drives the slider 2b and pushes out the second fluid from the ejection hole of the syringe 5 while the barrel 5a of the syringe 5 is fixed with the syringe clamp. The slider driver 2i may include, for example, an electric motor.

The syringe pump 2 includes a control unit 2k connected to the communication unit 2e, the power source 2f, the display 2d, the sound output unit 2g, the operation input unit 2h, the slider driver 2i, and a memory 2j. The control unit 2k can control the operations of, for example, the power source 2f, the slider driver 2i, the display 2d, and the sound output unit 2g according to input from the operation input unit 2h and a program stored in the memory 2j. The control unit 2k also controls the operation of the sensor unit 4a of the fluid sensor 4 via the communication unit 2e and the communication unit 4b.

The control unit 2k includes a notification control unit 21, a determination unit 2m, and a delivery control unit 2n. The notification control unit 21 is configured to cause the notification unit 2c to notify of the predetermined information when the communication unit 2e receives the first signal. The determination unit 2m is configured to determine whether the second fluid is the predetermined drug. The second fluid can be detected by, for example, configuring the syringe pump 2 so that the communication unit 2e reads information such as a product ID of the second fluid stored in a tag disposed in the syringe. Alternatively, the syringe pump 2 may be configured to be capable of acquiring the information such as the product ID of the second fluid from a library built in, for example, a server or a personal computer via the communication unit 2e. Alternatively, the syringe pump 2 may be configured so that the information such as the product ID of the second fluid is input by a user via the operation input unit 2h. The predetermined drug can be set by, for example, configuring the syringe pump 2 so that a user inputs information such as a product ID of the predetermined drug via the operation input unit 2h. The input information associated with the predetermined drug is stored in the memory 2j. The information associated with the predetermined drug may be stored in the memory 2j in advance without input from the user. The delivery control unit 2n is configured to be capable of controlling delivery of the second fluid to the infusion line 6. In this embodiment, when the determination unit 2m determines that the second fluid is the predetermined drug, the delivery control unit 2n is configured not to start delivering the second fluid to the infusion line 6 until the communication unit 2e receives the second signal. However, the delivery control unit 2n may be configured not to control initiation of the second fluid delivery.

The control unit 2k in this embodiment includes a central processing unit (CPU). Note that the control unit 2k may include, for example, a plurality of processing units.

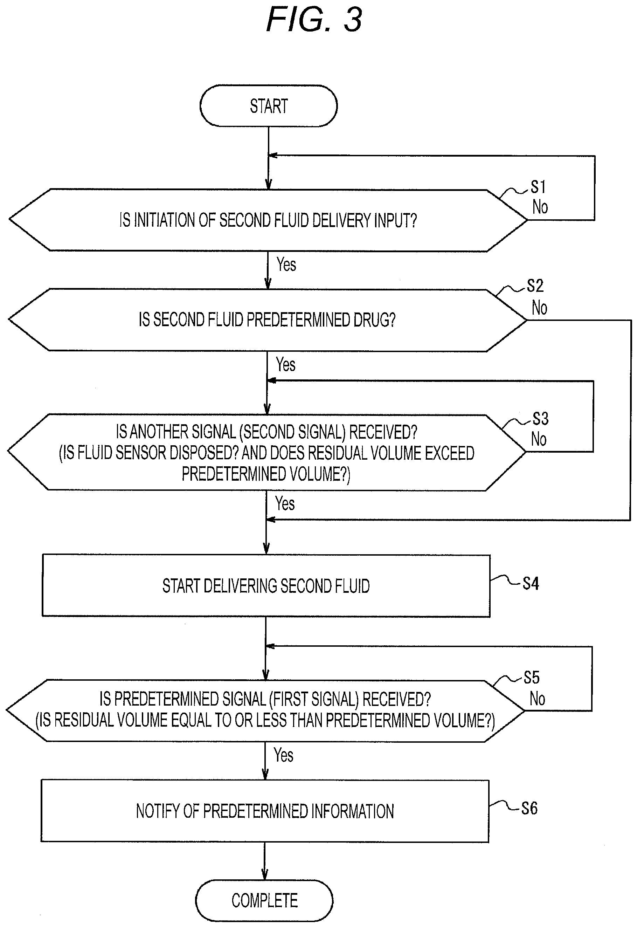

The infusion device 1 can operate in a manner illustrated in Fig. 3. First, in step S1, the control unit 2k determines whether initiation of the second fluid delivery is input to the operation input unit 2h. Determining that there is input ("Yes" in step S1), the control unit 2k proceeds to step S2. On the other hand, when the control unit 2k determines that there is no input ("No" in step S1), step S1 is repeated.

In step S2, the control unit 2k (determination unit 2m) determines whether the second fluid is the predetermined drug. Determining that the second fluid is the predetermined drug ("Yes" in step S2), the control unit 2k (determination unit 2m) proceeds to step S3. On the other hand, when the control unit 2k determines that the second fluid is not the predetermined drug ("No" in step S2), the control unit 2k proceeds to step S4.

In step S3, the control unit 2k (delivery control unit 2n) determines whether another signal (second signal) is received. Determining that the second signal is received ("Yes" in step S2), the control unit 2k (delivery control unit 2n) proceeds to step S4 and starts delivering the second fluid. On the other hand, when the control unit 2k determines that the second signal is not received ("No" in step S3), step S3 is repeated.

As described above, in this embodiment, when the second fluid is the predetermined drug such as an anesthetic, the second fluid is not delivered until it is determined that the second signal is received, that is, until the fluid sensor 4 is disposed in the infusion bag 3 and the residual volume of the first fluid L contained in the infusion bag 3 is determined to be over the predetermined volume. Accordingly, when the second fluid is the predetermined drug and when the attachment of the fluid sensor 4 to the infusion bag 3 is forgotten, it is possible to prevent the initiation of the second fluid delivery. In addition, when the second fluid is not the predetermined drug, it is possible to administer the second fluid to a living body without using the fluid sensor 4. In this case, the administering of the second fluid may be performed via the infusion line 6 or directly without the infusion line 6.

The control unit 2k may be configured not to perform the processes from step S2 to step S3. In this case, if "Yes" in step S1, the control unit 2k proceeds to step S4, skipping steps S2 and S3.

After the second fluid delivery is started in step S4, the control unit 2k proceeds to step S5. In step S5, the control unit 2k determines whether the predetermined signal (first signal) is received. Determining that the predetermined signal (first signal) is received ("Yes" in step S5), the control unit 2k proceeds to step S6 and notifies of the predetermined information. On the other hand, when the control unit 2k determines that the first signal is not received ("No" in step S5), step S5 is repeated.

In this manner, the control unit 2k can cause the notification unit 2c to notify of the predetermined information when the first signal is received, that is, when the residual volume inside the infusion bag 3 is equal to or less than the predetermined volume. In this embodiment, this notification is performed by the syringe pump 2 itself configured to administer the predetermined drug which may cause a serious situation when the infusion bag 3 is not replaced. Therefore, according to this embodiment, it is possible to urge a medical staff to replace the infusion bag 3, which reliably achieves the intended effect of the predetermined drug administered by the syringe pump 2.

The infusion device 1 and the syringe pump 2 according to this embodiment can be modified in various ways. For example, in this embodiment, the fluid sensor 4 is configured to be capable of transmitting the second signal when the residual volume of the first fluid L exceeds the predetermined volume, and the syringe pump 2 includes the determination unit 2m that determines whether the second fluid is the predetermined drug. When the determination unit 2m determines that the second fluid is the predetermined drug, the delivery control unit 2n is configured not to start delivering the second fluid to the infusion line 6 until the communication unit 2e receives the second signal. However, the syringe pump 2 may not include the determination unit 2m that determines whether the second fluid is the predetermined drug, and the delivery control unit 2n may be configured not to start delivering the second fluid to the infusion line 6 until the communication unit 2e receives the second signal.

Furthermore, the fluid sensor 4 may not be configured to be capable of transmitting the second signal when the residual volume of the first fluid L exceeds the predetermined volume. In this case, the determination unit 2m of the syringe pump 2 may be configured to determine whether the communication unit 2e and the fluid sensor 4 are communicably connected to each other and determine whether the second fluid is the predetermined drug. When the determination unit 2m determines that the second fluid is the predetermined drug, the delivery control unit 2n may be configured not to start delivering the second fluid to the infusion line 6 until the determination unit 2m determines that the communication unit 2e and the fluid sensor 4 are communicably connected to each other. The determination unit 2m of the syringe pump 2 may be configured to determine whether the communication unit 2e and the fluid sensor 4 are communicably connected to each other and not to determine whether the second fluid is the predetermined drug. The delivery control unit 2n may be configured not to start delivering the second fluid to the infusion line 6 until the determination unit 2m determines that the communication unit 2e and the fluid sensor 4 are communicably connected to each other.

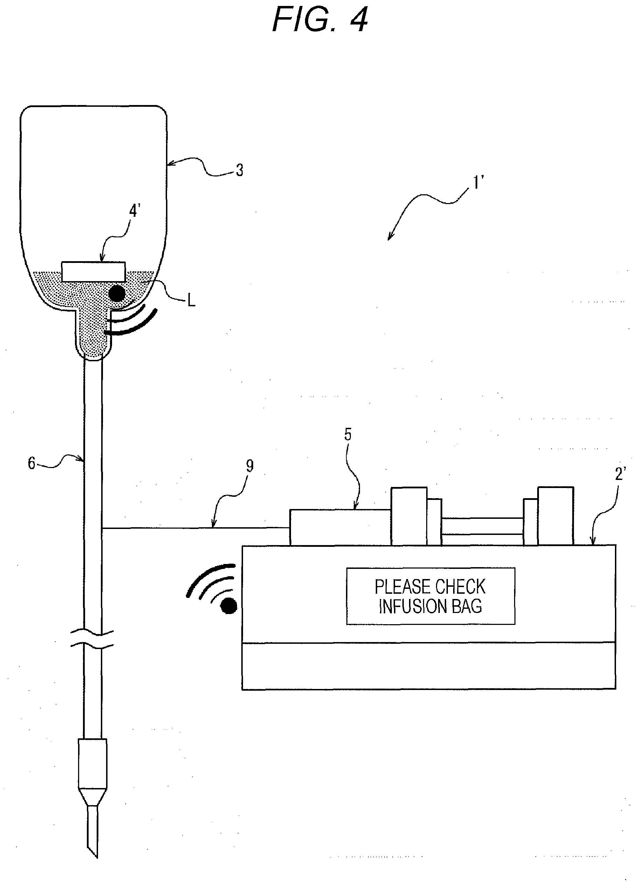

Next, an infusion device 1' and a syringe pump 2' according to a second embodiment of the present invention will be described in detail with reference to Figs. 4 to 5. As illustrated in Fig. 4, an infusion device 1' according to this embodiment is similar to the infusion device 1 according to the first embodiment except that a syringe pump 2' and a fluid sensor 4' are wirelessly connected to each other. In Figs. 4 to 5, components similar to those in the first embodiment are denoted by the same reference numerals as the first embodiment.

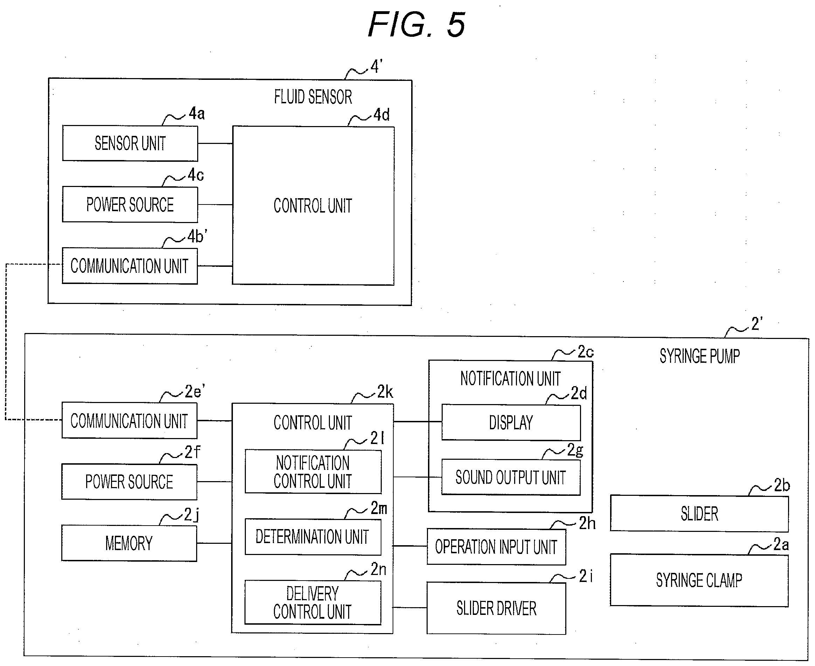

As illustrated in Fig. 5, in this embodiment, the fluid sensor 4' includes the sensor unit 4a, a communication unit 4b', a power source 4c, and a control unit 4d. The control unit 4d is connected to the sensor unit 4a, the communication unit 4b', and the power source 4c and configured to be capable of controlling operations of those members. The control unit 4d in this embodiment includes a microcontroller.

In this embodiment, a communication unit 2e' of the syringe pump 2' is configured to wirelessly be capable of communicating with the communication unit 4b' of the fluid sensor 4' by, for example, Bluetooth (registered trademark). In other words, the communication unit 2e' of the syringe pump 2' can receive by wireless communication either a first signal or a second signal from the communication unit 4b' of the fluid sensor 4' depending on the residual volume of a first fluid L. The other configurations of the syringe pump 2' are similar to those of the syringe pump 2 in the first embodiment.

The infusion device 1' can be operated in a similar manner to the infusion device 1 in the first embodiment. Therefore, the infusion device 1' according to the second embodiment can achieve effects similar to the infusion device 1 according to the first embodiment. The infusion device 1' according to the second embodiment can variously be modified in a similar manner to the infusion device 1 in the first embodiment.

The aforementioned embodiments are illustrated as exemplary embodiments of the present invention, and it goes without saying that the present invention may employ various modifications without departing from the gist of the invention.

### Reference Signs List

- 1, 1': Infusion device
- 2, 2': Syringe pump
- 2a: Syringe clamp
- 2b: Slider
- 2c: Notification unit
- 2d: Display
- 2e, 2e': Communication unit
- 2f: Power source
- 2g: Sound output unit
- 2h: Operation input unit
- 2i: Slider driver
- 2j: Memory
- 2k: Control unit
- 21: Notification control unit
- 2m: Determination unit
- 2n: Delivery control unit
- 3: Infusion bag
- 4, 4': Fluid sensor
- 4a: Sensor unit
- 4b, 4b': Communication unit
- 4c: Power source
- 4d: Control unit
- 5: Syringe
- 5a: Barrel
- 5b: Plunger
- 6: Infusion line
- 7: Indwelling member
- 8: Connecting member
- 9: Coinfusion line
- 10: Cable
- L: First fluid

## Claims

1. An infusion device comprising:
an infusion bag configured to contain a first fluid;
a syringe pump configured to hold a syringe and be capable of delivering a second fluid contained in the syringe to an infusion line connected to the infusion bag; and
a fluid sensor configured to be capable of transmitting a predetermined signal when a residual volume of the first fluid contained in the infusion bag is equal to or less than a predetermined volume,
wherein the syringe pump comprises:
a communication unit configured to be capable of receiving the predetermined signal from the fluid sensor;
a notification unit configured to be capable of notifying outside of predetermined information; and
a notification control unit configured to cause the notification unit to notify of the predetermined information when the communication unit receives the predetermined signal.

2. The infusion device according to claim 1,
wherein the syringe pump comprises:
a delivery control unit configured to be capable of controlling delivery of the second fluid to the infusion line; and
a determination unit configured to determine whether the communication unit and the fluid sensor are communicably connected to each other,
the delivery control unit being configured not to start delivering the second fluid to the infusion line until the determination unit determines that the communication unit and the fluid sensor are communicably connected to each other.

3. The infusion device according to claim 1,
wherein the syringe pump comprises:
a delivery control unit configured to be capable of controlling delivery of the second fluid to the infusion line; and
a determination unit configured to determine whether the communication unit and the fluid sensor are communicably connected to each other and determine whether the second fluid is a predetermined drug,
the delivery control unit being configured not to start delivering the second fluid to the infusion line until the determination unit determines that the communication unit and the fluid sensor are communicably connected to each other when the determination unit determines that the second fluid is the predetermined drug.

4. The infusion device according to claim 1,
wherein the fluid sensor is capable of transmitting another signal different from the predetermined signal when the residual volume of the first fluid exceeds the predetermined volume, and
the syringe pump includes a delivery control unit configured to be capable of controlling delivery of the second fluid to the infusion line,
the delivery control unit being configured not to start delivering the second fluid to the infusion line until the communication unit receives the other signal.

5. The infusion device according to claim 1,
wherein the fluid sensor is capable of transmitting another signal different from the predetermined signal when the residual volume of the first fluid exceeds the predetermined volume, and
the syringe pump comprises:
a determination unit configured to determine whether the second fluid is a predetermined drug; and
a delivery control unit configured to be capable of controlling delivery of the second fluid to the infusion line,
the delivery control unit being configured not to start delivering the second fluid to the infusion line until the communication unit receives the other signal when the determination unit determines that the second fluid is the predetermined drug.

6. The infusion device according to any one of claims 1 to 5, wherein the fluid sensor is an ultrasonic sensor disposed in the infusion bag.

7. A syringe pump configured to hold a syringe and be capable of delivering a second fluid contained in the syringe to an infusion line connected to an infusion bag that contains a first fluid, the syringe pump comprising:
a communication unit configured to be capable of receiving a predetermined signal from a fluid sensor when a residual volume of the first fluid contained in the infusion bag is equal to or less than a predetermined volume;
a notification unit configured to be capable of notifying outside of predetermined information; and
a notification control unit configured to cause the notification unit to notify of the predetermined information when the communication unit receives the predetermined signal.
